# FASCICULE DE BREVET EUROPEEN

(11) **EP 0 685 223 B1**
(45) Date de publication et mention de la délivrance du brevet: **21.08.1996**
(21) Numéro de dépôt: 95401057.5
(22) Date de dépôt: 05.05.1995
(51) Int. Cl.: A61K 7/42

(54) **Compositions cosmétiques anti-UV et utilisations**
Anti-UV kosmetische Mittel und Verwendungen
Anti-UV cosmetic compositions and uses

(30) Priorité: 03.06.1994 FR 9406831
(43) Date de publication de la demande: 06.12.1995
(73) Titulaire: L'OREAL, F-75008 Paris (FR)
(72) Inventeur: Hansenne, Isabelle, F-75017 Paris (FR); van Leeuwen, Alexandra Victoria, F-75020 Paris (FR)
(74) Mandataire: Andral, Christophe André Louis

(56) Documents cités:
- EP-A- 0 457 687
- EP-A- 0 521 651

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres lipophiles particuliers, l'un des filtres possédant des propriétés solubilisantes vis à vis de l'autre filtre. L'invention concerne par ailleurs un procédé de solubilisation d'un filtre lipophile solide particulier au moyen d'un ou deux autres filtres lipophiles liquides spécifiques.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV). Selon leur caractère lipophile ou au contraire hydrophile, ces filtres peuvent se répartir, respectivement, soit dans la phase grasse, soit dans la phase aqueuse, de la composition finale.

Il s'avère qu'un filtre particulièrement intéressant et largement utilisé à ce jour est constitué par la 2,4,6-tris[p-(2'-éthylhexyl-1'-oxycarbonyl)anilino]-1,3,5-triazine, qui est notamment vendu sous la dénomination commerciale de "UVINUL T 150" par la Société BASF.

Il s'agit d'un filtre lipophile, fortement actif dans l'UV-B, mais qui présente la particularité et aussi le désavantage d'être solide à température ambiante. De ce fait, son utilisation dans une composition cosmétique antisolaire implique certaines contraintes au niveau de sa formulation et de sa mise en oeuvre, en particulier lorsqu'il s'agit de trouver des solvants permettant de le solubiliser correctement. A cet égard, on fait aujourd'hui le plus souvent appel à des huiles comme des triglycérides et notamment des triglycérides d'acides gras en C₈-C₁₂ ("MIGLYOL 812" de chez Hüls), ou encore à des mono- ou des polyalcools gras oxyéthylénés ou oxypropylénés ("CETIOL HE" de chez Henkel, ou "WITCONOL APM" de chez Witco), ainsi qu'à leurs mélanges. Ces produits présentent néanmoins certains inconvénients, à savoir notamment que, d'une part, ils n'ont aucune activité propre (ou intrinsèque) dans le domaine de la filtration du rayonnement UV (UV-A et UV-B), et, d'autre part, qu'ils possédent des propriétés solubilisantes vis à vis du filtre susmentionné qui peuvent apparaître comme encore insuffisantes, tant pour ce qui touche aux limites de solubilité qu'aux vitesses de solubilisation de ce filtre dans ces solvants.

Or, la Demanderesse vient maintenant de découvrir, de façon inattendue et surprenante, que le salicylate d'homomenthyle (encore appelé plus simplement homosalate) et le salicylate d'octyle constituent tous deux, pris seuls ou en mélange, un très bon solvant pour la 2,4,6-tris[p-(2'-éthylhexyl-1'-oxycarbonyl)anilino]-1,3,5-triazine. En effet, et tout d'abord, ce dernier filtre présente dans ces deux premiers composés des solubilités relativement élevées, et en tous cas nettement supérieures à celles obtenues avec tous les autres solvants usuels utilisés à ce jour, ce qui permet, à quantité égale de solvant, de mettre en oeuvre des quantités plus importantes de filtre ; par ailleurs, la vitesse à laquelle la 2,4,6-tris[p-(2'-éthylhexyl-1'-oxycarbonyl)anilino]-1,3,5-triazine se solubilise dans les deux solvants susmentionnés est remarquablement améliorée, ce qui constitue bien entendu un avantage considérable à l'échelle d'une production industrielle (gains en temps et en énergie au niveau des mélanges, donc en rendement global, moindres coûts de production). On notera ici que les salicylates d'homomenthyle d'une part et d'octyle d'autre part sont des filtres lipophiles liquides déja connus pour leur activité dans l'UV-B, mais leurs propriétés solubilisantes vis à vis du filtre solide précité n'ont, quant à elles, jamais été décrites. Les intérêts de la présente découverte apparaissent ainsi multiples, en ce sens d'une part qu'il est maintenant possible non seulement d'effectuer la solubilisation de la 2,4,6-tris[p-(2'-éthylhexyl-1'-oxycarbonyl)anilino]-1,3,5-triazine par un solvant autre que ceux antérieurement connus, ce qui est toujours intéressant en soi, mais encore d'effectuer celle-ci de façon améliorée (solubilité et vitesse de solubilisation accrues) et tout ceci, d'autre part, en réalisant une augmentation substantielle, à concentration égale de ce filtre dans la composition antisolaire finale, du niveau de protection attachée à cette dernière.

Toutes ces découvertes sont à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine sous forme solubilisée à titre de filtre actif dans l'UV-B, et (ii) un système filtrant solubilisant dont les constituants sont choisis parmi le salicylate d'homomenthyle, le salicylate d'octyle et leurs mélanges. Selon un mode particulièrement préféré de réalisation de la présente invention, ledit système filtrant solubilisant est présent dans les compositions conformes à l'invention en une quantité suffisante pour solubiliser à lui seul l'ensemble dudit filtre UV-B.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

La présente invention a également enfin pour objet l'utilisation du salicylate d'homomenthyle et/ou du salicylate d'octyle pour solubiliser de la 2,4,6-tris[p-(2'-éthylhexyl-1'-oxycarbonyl)anilino]-1,3,5-triazine contenue dans une composition cosmétique antisolaire.

D'autres caractéristiques, aspects et avantages de la présente invention apparaitront à la lecture de la description détaillée qui va suivre.

Comme indiqué précédemment, la 2,4,6-tris[p-(2'-éthylhexyl-1'-oxycarbonyl)anilino]-1,3,5-triazine (composé A destiné à être solubilisé) est un filtre connu en soi, actif dans l'UV-B et se présentant sous une forme solide, qui est vendu notamment sous la dénomination commerciale de "UVINUL T 150" par la Société BASF. Ce produit répond à la formule (I) suivante: 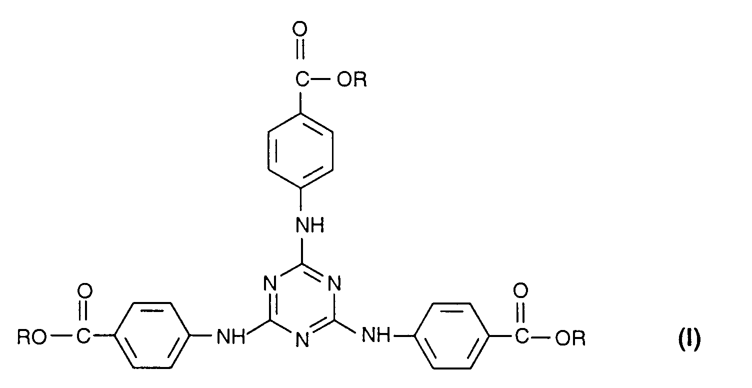 dans laquelle R désigne un radical 2-éthyl hexyle.

Concernant le salicylate d'homomenthyle (composé B destiné à solubiliser le composé A), connu aussi sous le nom d'homosalate, il s'agit également d'un produit disponible commercialement, et il est vendu notamment sous la dénomination de "KEMESTER HMS" par la Société Witco. Il répond à la formule (II) suivante:

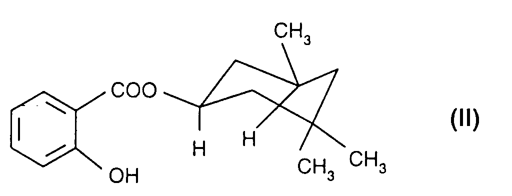

Enfin, le salicylate d'octyle (composé C destiné à solubiliser, conjointement ou non avec l'autre composé solubilisant B, le composés A) est, quant à lui, vendu notamment sous la dénomination commerciale de "UVINUL O-18" par la Société BASF, et il répond à la formule (III) suivante :

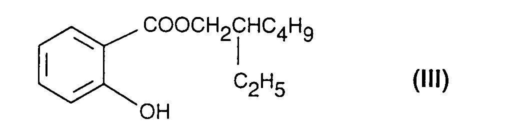

Le composé A (filtre UV-B à solubiliser) peut être présents dans les compositions selon l'invention à une concentration comprise entre 0,5 et 15 % en poids par rapport au poids total de la composition. Selon une caractéristique essentielle de la présente invention, ce composé doit se présenter dans la composition finale sous une forme totalement, ou substantiellement totalement, solubilisée.

Les composés B et/ou C (solubilisants) peuvent être, quant à eux, présents dans les compositions selon l'invention à des teneurs comprises entre 0,5 et 25 % en poids par rapport au poids total de la composition. Selon une caractéristique particulièrement préférée et avantageuse des compositions selon l'invention, ces composés sont utilisés, seuls ou en mélanges, en une quantité telle qu'elle soit suffisante pour solubiliser à elle seule la totalité, ou substantiellement la totalité, du composé A présent dans la composition. Dans ce dernier cas, cette quantité minimale en solvant(s) destinée à assurer une dissolution complète et stable du filtre solide peut être classiquement déterminée à partir de l'étude des paramètres de solubilité dudit filtre dans ces solvants.

A titre indicatif, il a été constaté que, à température ambiante, le composé A est soluble à raison de 16 % environ en poids dans le composé solvant B, et à raison de 25 % environ en poids dans le composé solvant C.

D'une manière générale, on notera que les concentrations en composés A, B et C, sont choisies de manière telle que l'indice de protection solaire de la composition finale soit de préférence d'au moins 2.

Selon une autre caractéristique particulièrement avantageuse des compositions conformes à l'invention, ces dernières ne contiennent de préférence aucun, ou substantiellement aucun, solubilisant pour le composé A autre que le ou les composés B et C précédemment définis. Selon l'invention, on considère qu'un composé donné ne possède pas de propriétés solubilisantes vis à vis d'un autre composé donné lorsque ce dernier composé présente une solubilité inférieure à 1 % en poids environ dans ce premier composé.

Enfin, toujours selon un mode préféré de réalisation de la présente invention, le support cosmétiquement acceptable dans lequel se trouvent contenus les différents composés A, B et/ou C, est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles autres, bien sûr, que les trois filtres lipophiles mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0 518 772 et EP-A-0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse représente généralement de 50 à 95 % en poids, de préférence de 70 à 90 % en poids, par rapport à l'ensemble de la formulation, la phase huileuse (comprenant les filtres lipophiles solubilisés et solubilisants) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation. On notera que, conformément à la présente invention, la phase grasse de telles émulsions peut n'être constituée pour l'essentiel ou même en totalité que des composés B et/ou C (solvants organiques) dans lesquels se trouve solubilisé le filtre A précédemment défini, ainsi que les éventuels filtres complémentaires et autres adjuvants cosmétiques lipophiles classiques.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

Dans cet exemple, on a déterminé le temps de solubilisation de la 2,4,6-tris[p-(2'-éthylhexyl-1'-oxycarbonyl)anilino]-1,3,5-triazine ("UVINUL T 150" de chez BASF) dans différents solvants au moyen du test suivant :
- à température ambiante, on ajoute 10 g de solvant à 5 g du filtre UVINUL T 150,
- on porte le mélange ainsi obtenu à 83° C au moyen d'un bain-marie,
- et on note alors le temps (en minutes) qui a été nécessaire pour solubiliser totalement le filtre après que cette température ait été atteinte.

Les résultats obtenus sont rassemblés dans le tableau ci-dessus (les noms chimiques sont donnés dans la nomenclature CTFA, 5ième édition, 1993).

**Tableau**

| SOLVANT | TEMPS DE SOLUBILISATION (mn) | |
|---|---|---|
| Octyl salicylate ("Uvinul O-18" de chez BASF | 0 | Invention |
| Homosalate ("Kemester HMS" de chez Witco) | 3 | |
| Triglycérides d'acides gras en C₈-C₁₂ ("MIGLYOL 812" de chez Hüls) | 10 | Comparatif |
| PEG-7 Glyceryl Cocoate ("Cetiol HE" de chez Henkel) | 10 | |
| PPG-3 Myristyl Ether ("Witconol APM" de chez Witco) | 15 | |

Ces résultas démontrent clairement le pouvoir solubilisant supérieur des deux solvants conformes à l'invention.

### EXEMPLE 2

On donne ici un exemple concret de formulation d'une composition conforme à l'invention sous la forme d'une émulsion de type huile-dans-eau.

| | |
|---|---|
| - 2,4,6-tris[p-(2'-éthylhexyl-1'-oxycarbonyl)anilino]-1,3,5-triazine ("UVINUL T 150") | 3 g |
| - Homosalate | 5 g |
| - Octyl salicylate | 10 g |
| - Polydiméthylsiloxane | 1,5 g |
| - Alcool cétylique | 1,5 g |
| - Huile de vaseline | 5 g |
| - Mélange mono/di-stéarate de glycérine | 2 g |
| - Mélange d'alcools cétylstéarylique et cétylstéarylique oxyéthyléné à 33 moles d'oxyde d'éthylène (80%/20%) ("Cire de Lanol CTO" de chez Seppic) | 7 g |
| - Glycérine | 4 g |
| - Oxyde de titane nanopigmentaire | 2 g |
| - Conservateurs | qs |
| - Eau | qsp 100 g |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine (composé A) sous forme solubilisée à titre de filtre actif dans l'UV-B, et (ii) un système filtrant solubilisant dont les constituants sont choisis parmi le salicylate d'homomenthyle (composé B), le salicylate d'octyle (composé C) et leurs mélanges.

2. Compositions selon la revendication 1, caractérisées par le fait que ledit système filtrant solubilisant est présent en une quantité suffisante pour solubiliser à lui seul l'ensemble du composé A.

3. Compositions selon la revendication 1 ou 2, caractérisées par le fait que le composé A est présent à raison de 0,5 à 15 % en poids par rapport à l'ensemble de la composition.

4. Compositions selon l'une quelconque des revendications précédentes, caractérisées par le fait que ledit système filtrant solubilisant est présent à raison de 0,5 à 25 % en poids par rapport à l'ensemble de la composition.

5. Compositions selon l'une quelconque des revendications précédentes, caractérisées par le fait qu'elles sont exemptes ou substantiellement exemptes d'un agent solubilisant pour le composé A autre que le ou les composés B et C.

6. Compositions selon l'une quelconque des revendications précédentes, caractérisées par le fait que ledit support cosmétiquement acceptable se présente sous la forme d'une émulsion de type huile-dans-eau.

7. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

8. Compositions selon la revendication 7, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres

9. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

10. Compositions selon la revendication 9, caractérisées par le fait que lesdits pigments ou nanopigments sont choisis parmi les oxydes de titane, de zinc, de fer, de zirconium, de cérium et leurs mélanges, enrobés ou non.

11. Compositions selon l'une quelconque des revendications précédentes, caractérisées par le fait qu'elles comprennent en outre au moins un agent de bronzage et/ou de brunissage artificiel de la peau.

12. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

13. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

14. Compositions selon l'une quelconque des revendications 1 à 12, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

15. Compositions selon l'une quelconque des revendications 1 à 12, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

16. Compositions selon l'une quelconque des revendications précédentes, caractérisées par le fait qu'elles présentent un indice de protection sur peau d'au moins 2.

17. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

18. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 16.

19. Utilisation du salicylate d'homomenthyle et/ou du salicylate d'octyle pour solubiliser de la 2,4,6-tris[p-(2'-éthylhexyl-1'-oxycarbonyl)anilino]-1,3,5-triazine contenue dans une composition cosmétique antisolaire.

## Claims

1. Cosmetic compositions for topical use, in particular for the photoprotection of the skin and/or hair, characterized in that they comprise, in a cosmetically acceptable vehicle, (i) 2,4,6-tris[p-(2'-ethylhexyl-1'-oxycarbonyl)anilino]-1,3,5-triazine (compound A) in the dissolved form, as screening agent active in the UV-B, and (ii) a solubilizing screening system, the constituents of which are chosen from homomenthyl salicylate (compound B), octyl salicylate (compound C) and their mixtures.

2. Compositions according to Claim 1, characterized in that the said solubilizing screening system is present in an amount sufficient to dissolve, on its own, the whole of the compound A.

3. Compositions according to Claim 1 or 2, characterized in that the compound A is present in a proportion of 0.5 to 15 % by weight with respect to the whole of the composition.

4. Compositions according to any one of the preceding claims, characterized in that the said solubilizing screening system is present in a proportion of 0.5 to 25 % by weight with respect to the whole of the composition.

5. Compositions according to any one of the preceding claims, characterized in that they are free or substantially free from a solubilizing agent for the compound A, other than the compound(s) B and C.

6. Compositions according to any one of the preceding claims, characterized in that the said cosmetically acceptable vehicle is provided in the form of an emulsion of oil-in-water type.

7. Compositions according to any one of the preceding claims, characterized in that they additionally comprise one or a number of additional, hydrophilic or lipophilic, organic screening agents which are active in the UV-A and/or UV-B.

8. Compositions according to Claim 7, characterized in that the said additional organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives or polymer screening agents and silicone screening agents.

9. Compositions according to any one of the preceding claims, characterized in that they additionally comprise, as additional photoprotective agents, coated or non-coated metal oxide pigments or nanopigments capable of physically blocking, by scattering and/or reflection, the UV radiation.

10. Compositions according to Claim 9, characterized in that the said pigments or nanopigments are chosen from coated or non-coated titanium oxide, zinc oxide, iron oxide, zirconium oxide, cerium oxide and their mixtures.

11. Compositions according to any one of the preceding claims, characterized in that they additionally comprise at least one artificial bronzing and/or tanning agent for the skin.

12. Compositions according to any one of the preceding claims, characterized in that they additionally comprise at least one adjuvant chosen from fatty substances, organic solvents, ionic or non-ionic thickeners, softeners, antioxidants, opacifying agents, stabilizers, emollients, silicones, α-hydroxy acids, anti-foaming agents, moisturizing agents, vitamins, fragrances, preservatives, surface-active agents, fillers, sequestering agents, polymers, propellants, basifying or acidifying agents or dyes.

13. Compositions according to any one of the preceding claims, characterized in that it concerns protective compositions for the human skin or anti-sun compositions and in that they are provided in the form of non-ionic vesicular dispersions or emulsions, in particular emulsions of oil-in-water type, creams, milks, gels, cream gels, suspensions, dispersions, powders, solid sticks, foams or spray.

14. Compositions according to any one of Claims 1 to 12, characterized in that it concerns make-up compositions for the eyelashes, eyebrows or skin and in that they are provided in the form, anhydrous or aqueous, solid or pasty, of emulsions, suspensions or dispersions.

15. Compositions according to any one of Claims 1 to 12, characterized in that it concerns compositions intended for the protection of the hair against ultraviolet radiation and in that they are provided in the form of shampoos, lotions, gels, emulsions, non-ionic vesicular dispersions or hair lacquers.

16. Compositions according to any one of the preceding claims, characterized in that they have a protection factor on the skin of at least 2.

17. Use of the compositions defined in any one of the preceding claims as, or for the manufacture of, cosmetic compositions for the protection of the skin and/or hair against ultraviolet radiation and in particular solar radiation.

18. Cosmetic treatment process for protecting the skin and/or hair against ultraviolet radiation, in particular solar radiation, characterized in that it consists in applying an effective amount of a composition as defined in any one of Claims 1 to 16 to the latter.

19. Use of homomenthyl salicylate and/or of octyl salicylate for dissolving 2,4,6-tris[p-(2'-ethylhexyl-1'-oxycarbonyl)anilino]-1,3,5-triazine contained in an anti-sun cosmetic composition.

## Patentansprüche

1. Kosmetische Zusammensetzungen zur topischen Anwendung, insbesondere zum Lichtschutz der Haut und/oder der Haare,
dadurch gekennzeichnet, daß
sie in einem kosmetisch akzeptablen Träger (i) 2,4,6-Tris[p-(2'-ethylhexyl-1'-oxycarbonyl)anilino]-1,3,5-triazin (Verbindung A) in solubilisierter Form als im UV-B-Bereich wirksames Filter und (ii) ein solubilisierendes Filtersystem, dessen Bestandteile unter Homomenthylsalicylat (Verbindung B), Octylsalicylat (Verbindung C) und deren Gemischen ausgewählt sind, enthalten.

2. Zusammensetzungen nach Anspruch 1, dadurch gekennzeichnet, daß das solubilisierende Filtersystem in einer Menge enthalten ist, die allein ausreicht, die gesamte Verbindung A zu solubilisieren.

3. Zusammensetzungen nach Anspruch 1 oder 2, dadurch gekennzeichnet, daß die Verbindung A in einem Anteil von 0,5 bis 15 Gew.-%, bezogen auf die gesamte Zusammensetzung, enthalten ist.

4. Zusammensetzungen nach einem der vorhergehenden Ansprüche, dadurch gekennzeichnet, daß das solubilisierende Filtersystem in einem Anteil von 0,5 bis 25 Gew.-%, bezogen auf die gesamte Zusammensetzung, enthalten ist.

5. Zusammensetzungen nach einem der vorhergehenden Ansprüche, dadurch gekennzeichnet, daß sie frei oder im wesentlichen frei von solubilisierenden Mitteln für die Verbindung A sind, die von der oder den Verbindungen B und C verschieden sind.

6. Zusammensetzungen nach einem der vorhergehenden Ansprüche, dadurch gekennzeichnet, daß der kosmetisch akzeptable Träger in Form einer Öl-in-Wasser-Emulsion vorliegt.

7. Zusammensetzungen nach einem der vorhergehenden Ansprüche, dadurch gekennzeichnet, daß sie ferner ein oder mehrere ergänzende hydrophile oder lipophile organische Filter, die im UV-A-Bereich und/oder UV-B-Bereich wirksam sind, enthalten.

8. Zusammensetzungen nach Anspruch 7, dadurch gekennzeichnet, daß die ergänzenden organischen Filter unter Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, polymeren Filtern und Siliconfiltern ausgewählt sind.

9. Zusammensetzungen nach einem der vorhergehenden Ansprüche, dadurch gekennzeichnet, daß sie ferner als ergänzende Lichtschutzmittel gegebenenfalls umhüllte Pigmente oder Nanopigmente von Metalloxiden enthalten, die die UV-Strahlung physikalisch durch Streuung und/oder Reflexion zu sperren vermögen.

10. Zusammensetzungen nach Anspruch 9, dadurch gekennzeichnet, daß die Pigmente oder Nanopigmente unter ggf. umhüllten Titanoxiden, Zinkoxiden, Eisenoxiden, Zirconiumoxiden, Ceroxiden und deren Gemischen ausgewählt sind.

11. Zusammensetzungen nach einem der vorhergehenden Ansprüche, dadurch gekennzeichnet, daß sie außerdem mindestens ein Bräunungsmittel und/oder ein Mittel zur künstlichen Hautbräunung enthalten.

12. Zusammensetzungen nach einem der vorhergehenden Ansprüche, dadurch gekennzeichnet, daß sie ferner mindestens einen Zusatzstoff enthalten, der unter Fettsubstanzen, organischen Lösungsmitteln, ionischen oder nichtionischen Verdickungsmitteln, Mitteln für die Geschmeidigkeit, Antioxidationsmitteln, Trübungsmitteln, Stabilisatoren, Softenern, Siliconen, α-Hydroxysäuren, Antischaummitteln, Hydratisierungsmitteln, Vitaminen, Parfums, Konservierungsmitteln, grenzflächenaktiven Stoffen, Füllstoffen, Maskierungsmitteln, Polymeren, Treibmitteln, alkalisch oder sauer machenden Mitteln und Färbemitteln ausgewählt sind.

13. Zusammensetzung nach einem der vorhergehenden Ansprüche, dadurch gekennzeichnet, daß es sich um die menschliche Haut schützende Zusammensetzungen oder Sonnenschutzzusammensetzungen handelt und daß sie in Form von Bispersionen nichtionischer Vesikel, in Form von Emulsionen, insbesondere Öl-in-Wasser-Emulsionen, Cremes, Milchen, Gelen, Gelcremes, Suspensionen, Dispersionen, Pudern, festen Stiften, Schäumen oder Sprays, vorliegen.

14. Zusammensetzungen nach einem der Ansprüche 1 bis 12, dadurch gekennzeichnet, daß es sich um Zusammensetzungen zum Schminken der Wimpern, der Augenbrauen oder der Haut handelt und daß sie in fester oder pastöser, wasserfreier oder wäßriger Form, als Emulsionen, Suspensionen oder Dispersionen vorliegen.

15. Zusammensetzungen nach einem der Ansprüche 1 bis 12, dadurch gekennzeichnet, daß es sich um Zusammensetzungen handelt, die für den Schutz der Haare gegen UV-Strahlung bestimmt sind, und daß sie in Form von Shampoos, Lotionen, Gelen, Emulsionen, Dispersionen nichtionischer Vesikel, Haarsprays oder Haarlacken vorliegen.

16. Zusammensetzungen nach einem der vorhergehenden Ansprüche, dadurch gekennzeichnet, daß sie einen Lichtschutzfaktor auf der Haut von mindestens 2 aufweisen.

17. Verwendung der in den vorhergehenden Ansprüchen definierten Zusammensetzungen als kosmetische Zusammensetzungen oder für die Herstellung von kosmetischen Zusammensetzungen für den Schutz der Haut und/oder der Haare gegen UV-Strahlung und insbesondere gegen Sonnenlicht.

18. Verfahren zur kosmetischen Behandlung zum Schutz der Haut und/oder der Haare gegen UV-Strahlung und insbesondere Sonnenlicht, dadurch gekennzeichnet, daß es darin besteht, eine wirksame Menge einer in den Ansprüchen 1 bis 16 definierten Zusammensetzung auf die Haut und/oder das Haar aufzutragen.

19. Verwendung von Homomenthylsalicylat und/oder Octylsalicylat zur Solubilisierung von 2,4,6-Tris[p-(2'-ethylhexyl-1'-oxycarbonyl)anilino]-1,3,5-triazin, das in einer kosmetischen Lichtschutzzusammensetzung enthalten ist.
